Europäisches Patentamt

European Patent Office

Office européen des brevets

(19)

(11) **EP 1 370 188 B1**

(12) **FASCICULE DE BREVET EUROPEEN**

(45) Date de publication et mention
de la délivrance du brevet:
**09.08.2006 Bulletin 2006/32**

(21) Numéro de dépôt: **02716893.9**

(22) Date de dépôt: **14.03.2002**

(51) Int Cl.:
*A61B 19/00* *(2006.01)*

(86) Numéro de dépôt international:
**PCT/FR2002/000912**

(87) Numéro de publication internationale:
**WO 2002/074177 (26.09.2002 Gazette 2002/39)**

(54) **PROCEDE ET SYSTEME DE RECONSTRUCTION A DISTANCE D'UNE SURFACE**

VERFAHREN UND SYSTEM ZUR FERNGESTEUERTEN FLÄCHENREKONSTRUKTION

METHOD AND SYSTEM FOR REMOTE RECONSTRUCTION OF A SURFACE

(84) Etats contractants désignés:
**AT BE CH CY DE DK ES FI FR GB GR IE IT LI LU
MC NL PT SE TR**

(30) Priorité: **21.03.2001 FR 0103828**

(43) Date de publication de la demande:
**17.12.2003 Bulletin 2003/51**

(73) Titulaire: **FRANCE TELECOM
75015 Paris (FR)**

(72) Inventeurs:
• **HENNION, Bernard
F-38410 Saint Martin d'Uriage (FR)**
• **GUERRAZ, Agnès
F-38450 Le Gua (FR)**

(74) Mandataire: **Loisel, Bertrand et al
Cabinet Plasseraud
65/67 rue de la Victoire
75440 Paris Cedex 09 (FR)**

(56) Documents cités:
**EP-A- 0 411 498          US-A- 5 800 178**

## Description

La présente invention relève du domaine de la reconstruction à distance d'une surface, notamment d'une surface gauche.

Pour la transmission de retour d'effort à distance, notamment dans le cadre du développement de mondes virtuels répartis et du développement de moyens de téléprésence, le besoin apparaît de déterminer les coordonnées d'une surface éloignée d'un moyen de calcul ou de commande, et ce dans un but d'étalonnage.

Les méthodes classiques, notamment d'interpolation, de lissage, de Lagrange, de Hermite..., utilisent généralement des données de position dans l'espace prises judicieusement pour obtenir un résultat proche du modèle réel. Les calculs qui en découlent sont lourds et ne peuvent guère être effectués en temps réel. La qualité du résultat dépend du nombre de points connus de l'objet réel, de la pertinence des points mesurés et de la quasi homothétie des modèles virtuel et réel.

De la même façon que les techniques de codage de vidéo numérique s'appuient sur les connaissances de la perception visuelle, et les techniques de réconnaissance vocale sur les connaissances de l'ouïe, les techniques haptiques (du grec haptos : la main) s'appuient sur les connaissances des gestes.

Il existe deux sortes de gestes fins réalisables avec la main : les gestes balistiques, comme déplacer la main vers un verre pour le saisir et les gestes avec contre-réactions du toucher, comme celui qui permet après saisie du verre et fermeture de la pince pouce-doigts, de porter le verre à sa bouche. Le cerveau est alors informé en permanence de la force avec laquelle la main enserre le verre, de son poids qui dépend de la quantité de liquide. Le cerveau réagit alors en donnant l'ordre moteur de "pincer" suffisamment ce verre pour qu'il ne tombe pas, mais pas trop toutefois, pour ne pas le casser, ni dépenser une énergie inutile.

Les gestes balistiques activent l'arc moteur mais ils n'activent pas l'arc sensitif en retour du toucher. Le retour d'informations peut être une représentation visuelle de l'espace, mais aussi une carte gestuelle, c'est-à-dire une représentation mentale apprise ou innée câblée dans le cerveau et qui génère automatiquement la séquence des ordres moteurs des muscles de l'épaule, du bras, de la main pour réaliser ce geste balistique en fonction d'une certaine représentation mentale de l'espace, notamment de la distance présumée main-verre.

Pour les gestes balistiques, il est suffisant de transmettre au cerveau les informations sur le geste avec une fréquence d'échantillonnage de 100 Hz. Cela signifie que si on envoie un échantillon du signal toutes les 10 ms, le signal transmis contiendra toute l'information pertinente du geste balistique.

Les gestes avec contre-réactions du toucher activent en même temps l'arc moteur et l'arc sensitif. Le cerveau ferme la boucle et le cycle complet chez l'homme dure moins de 1 ms. La bande passante des neurones sensitifs situés dans les bouts des doigts, c'est-à-dire la fréquence maximale du signal mécanique que ces neurones sont capables de détecter et de transmettre au cerveau, est supérieure à 500 Hz. Si on veut pouvoir coder dans un ordinateur un geste fin, il faut que le système à retour d'efforts utilisé ait lui-même une fréquence de fonctionnement élevée, au moins égale, d'après le théorème de Shannon, au double de la bande passante des doigts.

Dans la pratique, les systèmes à retour d'efforts sur une machine locale fonctionnent à une fréquence typique de 1 KHz en boucle fermée locale, c'est-à-dire qu'une rétroaction est calculée puis exercée sur leurs moteurs puis perçue par la main toutes les 1/1000 de seconde. Cela permet d'éviter l'effet dit de la "brosse à dents électrique" : l'instrument que l'on tient en main ne doit pas donner l'impression de vibrer.

Cette fréquence de 1 KHz résulte du compromis suivant : elle ne doit être ni trop basse pour pouvoir restituer finement l'impression tactile, ni trop haute pour laisser suffisamment de temps à l'ordinateur pour calculer la force de contre-réaction qui va représenter, dans le monde virtuel mécanique, la simulation fine du geste effectué.

Si maintenant on désire transmettre via un réseau de télécommunications, des gestes fins codés par le système à retour d'efforts et des gestes fins avec contre-réactions, le problème se complique du fait de la latence généralement bien supérieure introduite par le réseau lui-même.

Ainsi, la latence en technologie RNIS est de 30 ms, en technologie ADSL de l'ordre de 200 ms, et sur Internet elle peut atteindre 6 s ou même provoquer le rejet pur et simple du message. Sur ADSL et Internet, la latence varie du fait de la nature asynchrone des réseaux. La cadence de 1 KHz est donc beaucoup trop élevée pour pouvoir être maintenue si la boucle fermée inclut un aller-retour via le réseau - le geste est codé puis transmis via le réseau, il est appliqué à un objet distant, la contre-réaction de cet objet est à son tour codée et transmise en retour via le réseau.

Un geste balistique peut être transmis avec un retard de l'ordre de grandeur de 10 ms. En effet, la vue est un sens monodirectionnel : l'oeil est une sorte de caméra enregistrant une scène et le cerveau, à une tolérance près, peut percevoir avec un léger retard le film visuel précis sans perturber l'exécution du geste.

Au contraire, un geste fin avec contre-réaction nécessite de boucler en moins d'une milliseconde, l'aller-retour de décision de l'intensité de la force à exercer :

- émission de l'ordre au muscle via l'arc sensitif moteur,
- action mécanique de la main sur le verre,

- sensation du toucher du verre (augmentation de la pression de contact) au niveau des bouts des doigts, et
- retour vers le cerveau via l'arc sensitif tactile de cette information pour permettre au cerveau de décider l'ajustement de la force de la "pince".

Pour tenter de transmettre néanmoins un tel geste fin, il existe une méthode dite Méthode "Wavetransform", publiée par John Wilson, Neville Hogan du MIT sous le titre "Algorithms for Network - B ased Force Feedback", Fourth PHANTOM Users Group Workshop (PUG 99). Cette méthode simule le retard introduit par le réseau par une viscosité artificielle qui stabilise la boucle de contre-réaction : le système est d'autant plus visqueux que le réseau introduit un grand retard.

La méthode "Wavetransform" consiste à transposer dans l'espace forces/vitesses la théorie des quadripôles passifs à retard pur qui est bien connue pour les grandeurs électriques tension/intensité. Cette théorie permet de calculer les ondes électriques incidentes et réfléchies en fonction de l'impédance caractéristique de la ligne. La transmission du signal électrique est optimale lorsque cette ligne est fermée sur cette même valeur caractéristique de l'impédance.

La loi d'Ohm U=Z*I se transpose dans l'espace mécanique en la loi F = viscosité * Vitesse et la méthode "Wavetransform" consiste à adapter une ligne à retard pure virtuelle en lui donnant comme impédance (viscosité en fait) caractéristique celle du robot télémanipulé. Le signal est transmis sous la forme de sa transformée en Z, $S(z) = \Sigma$ $(s(t)*e^{(2i*\pi*n*T)})$ où T est le délai fixe du réseau. Plus le réseau introduit un grand retard, et plus il faut rajouter une grande viscosité artificielle dans la ligne pour stabiliser la simulation mécanique répartie du geste fin en boucle fermée et en réseau.

On déforme certes la sensation gestuelle, mais on optimise la transmission du signal utile. Cette méthode a été publiée au Fourth Users Group Workshop (PUG99).

La méthode "Wavetransform" nécessite un réseau synchrone, c'est-à-dire un réseau à retard fixe et connu, par exemple RNIS. Elle est basée sur la représentation en Z des signaux discrets échantillonnés de période égale à ce délai fixe connu du réseau.

Elle est donc inapplicable sur les réseaux asynchrones à messages du type Internet ou ATM, UMTS, qui se caractérisent par un délai de transmission variable, et un rejet si le message se perd ou met trop de temps à traverser le réseau.

Le problème de la cadence trop élevée des systèmes à retours d'efforts est exacerbé sur ces réseaux asynchrones, pour lesquels :

- les messages peuvent se perdre, ne pas aboutir, ou être rejetés si l'accusé de réception tarde trop (TCP/IP),
- les messages qui arrivent à bon port mettent un délai variable pour traverser le réseau,
- ils n'arrivent pas forcément dans l'ordre où ils ont été émis,
- il n'existe pas d'horloge commune exacte à la milliseconde près entre deux machines.

EP-A 411 498 décrit un procédé de guidage à distance d'un élément mobile à l'aide d'un environnement simulé.

Le modèle de l'environnement dans lequel l'élément mobile évolue, est comparé aux images transmises par une caméra portée par cet élément mobile, ce qui permet de corriger le modèle.

L'invention propose de remédier aux inconvénients des systèmes de l'art antérieur.

L'invention propose de reconstruire à distance une surface locale pour pouvoir calculer une consigne dans un délai court.

L'invention propose, notamment, un système de commande d'un élément à rétroaction situé à distance susceptible de fonctionner avec transmission de données sur réseaux synchrones ou asynchrones, de retard connu ou indéterminé.

Le procédé de reconstruction à distance d'une surface, selon un aspect de l'invention, comprend les étapes suivantes :

- un système local envoie à un système distant une information de position d'un élément mobile dudit système local, l'élément mobile du système local répliquant la position de l'élément mobile du système distant, le système distant comprenant une modélisation distante de la surface,
- un opérateur distant déplace l'élément mobile du système distant, et lorsque l'élément mobile du système local entre en contact avec ladite surface, ladite modélisation distante est modifiée pour chaque point de contact entre l'élément mobile du système local et ladite surface de façon que la modélisation distante se rapproche de ladite surface. On effectue ainsi un étalonnage qui permet que le modèle distant simule de façon précise la surface locale.

Dans un mode de réalisation de l'invention, la modélisation distante est initialement une surface plane maillée. La modélisation distante peut être initialement un ensemble plan d'éléments triangulaires contigus.

Dans un mode de réalisation de l'invention, la modélisation distante est rapprochée dans son ensemble de ladite surface selon un mouvement de translation normale à une portion de ladite surface normale à une portion de la modélisation distante, jusqu'à un premier point de contact de l'élément mobile du système local avec ladite surface.

Les coordonnées dudit premier point de contact peuvent être prédéterminées selon deux axés d'un repère tridimensionnel, seule étant à déterminer la coordonnée selon un troisième axe dudit repère tridimensionnel.

Dans un mode de réalisation de l'invention, un point de contact est un point de l'espace commun à l'élément mobile du système local et à ladite surface et tel que l'élément mobile du système local exerce sur ladite surface une force prédéterminée. Généralement, ladite force sera normale à ladite surface.

Avantageusement, la modélisation distante comprend une pluralité de noeuds, un noeud est rapproché de ladite surface par translation selon un axe. L'axe de translation peut être parallèle audit troisième axe. De façon alternative, un noeud peut être rapproché de ladite surface par rotation autour d'un noeud adjacent avec conservation de la distance entre lesdits deux noeuds.

Dans un mode de réalisation de l'invention, la force de réaction exercée par ladite surface sur l'élément mobile du système local est répliquée par l'élément mobile du système distant de façon que l'opérateur perçoive lesdites forces de réaction et puisse appréhender ladite surface.

Avantageusement, lors d'une phase d'initialisation, l'élément mobile du système distant réplique la position de l'élément mobile du système local.

Dans un mode de réalisation de l'invention, l'élément mobile du système distant exerce une force de freinage lorsque l'élément mobile du système local est déplacé vers ladite surface dans une partie de l'espace comprise entre la modélisation et ladite surface. L'opérateur perçoit ainsi ladite force de freinage et peut appréhender ladite modélisation.

L'invention propose également un système de reconstruction à distance d'une surface. Le système comprend un système local pourvu d'un élément mobile apte à entrer en contact avec la surface à reconstruire et un système distant pourvu d'un élément mobile apte à être manipulé par un opérateur, d'une modélisation distante de la surface et d'un moyen pour modifier ladite modélisation distante pour chaque point de contact entre l'élément mobile du système local et ladite surface lorsque l'élément mobile du système local entre en contact avec ladite surface, de façon que la modélisation distante se rapproche de ladite surface. Le système local est pourvu d'un moyen pour envoyer au système distant une information de position de l'élément mobile dudit système local, et d'un moyen pour répliquer la position de l'élément mobile du système distant.

L'invention concerne également un programme d'ordinateur comprenant des moyens de code programme pour mettre en oeuvre les étapes du procédé, lorsque ledit programme fonctionne sur un ordinateur.

L'invention concerne également un support capable d'être lu par un dispositif de lecture de moyens de code programme qui s'y trouvent stockés et qui sont aptes à la mise en oeuvre des étapes du procédé, lorsque ledit programme fonctionne sur un ordinateur.

On peut ainsi modéliser et étalonner un objet ou une forme tridimensionnelle distant de forme totalement libre. Le flux de données haptiques et utilisé pour transmettre le maximum de données sur ladite forme distante.

La présente invention s'applique de façon avantageuse à des systèmes bidirectionnels, par exemple la télé-échographie robotisée qui peut être utilisée dans le domaine de l'obstétrique et des examens abdominaux. On peut télé-calibrer un mannequin virtuel 3D d'une personne.

Dans le cas de l'échographie, la peau de la personne est généralement enduite d'un gel pour une transmission convenable des ultra sons. La sonde échographique pourra être manipulée à distance par un opérateur. En raison de la présence du gel, les composantes de force exercée par le patient ou la patiente sur la sonde peuvent être considérées comme normales à la surface locale de la peau. La sonde possède 6 degrés de liberté avec une contre réaction de force selon les trois axes d'un repère tridimentionnel et une contre réaction de couple également selon les trois axes d'un repère tridimensionnel.

Le système peut également être mis à profit par des personnes ayant une vue déficiente pour appréhender une forme.

Le système peut également s'appliquer à des applications industrielles du genre télé-usinage, télé-commande de robots en atmosphère hostile à l'homme ou sous faible visibilité. Le système est bien adapté pour effectuer des palpations à distance.

La présente invention sera mieux comprise et d'autres avantages apparaîtront à la lecture de la description détaillée de quelques modes de réalisation pris à titre d'exemple nullement limitatifs et illustrés par les dessins annexés, sur lesquels :

la figure 1 est une vue schématique d'un système selon un mode de réalisation de l'invention;
la figure 2 est une vue détaillée des systèmes S 1 et S2 de la figure 1;
la figure 3 est une vue plus détaillée de la figure 2;

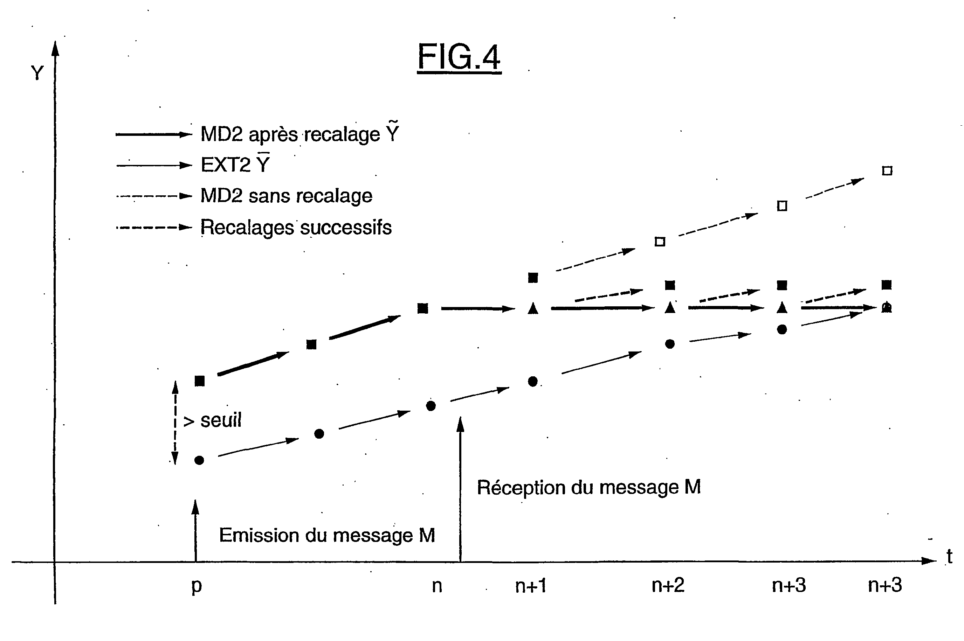

la figure 4 montre des courbes de recalage;
la figure 5 est une vue schématique d'un système selon un mode de réalisation de l'invention;
la figure 6 est une vue schématique de la reconstruction en un point; et

la figure 7 est un organigramme des étapes du procédé.

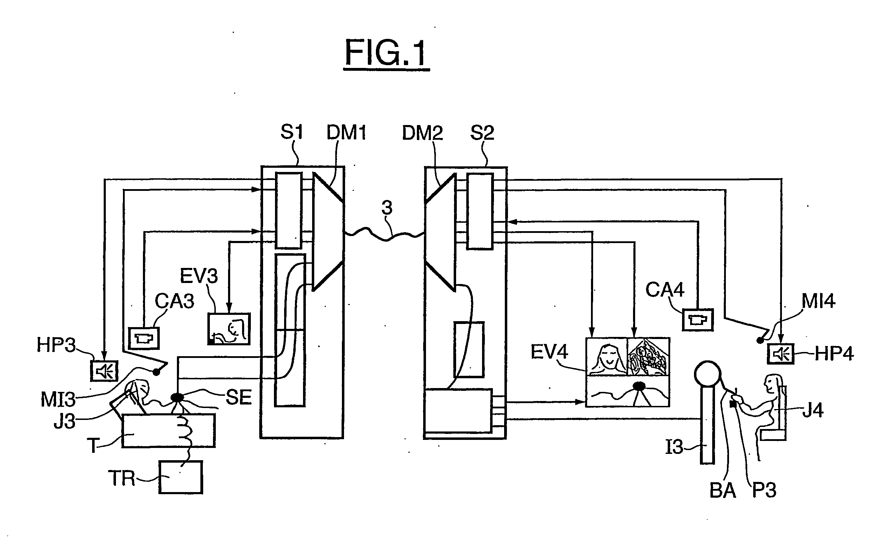

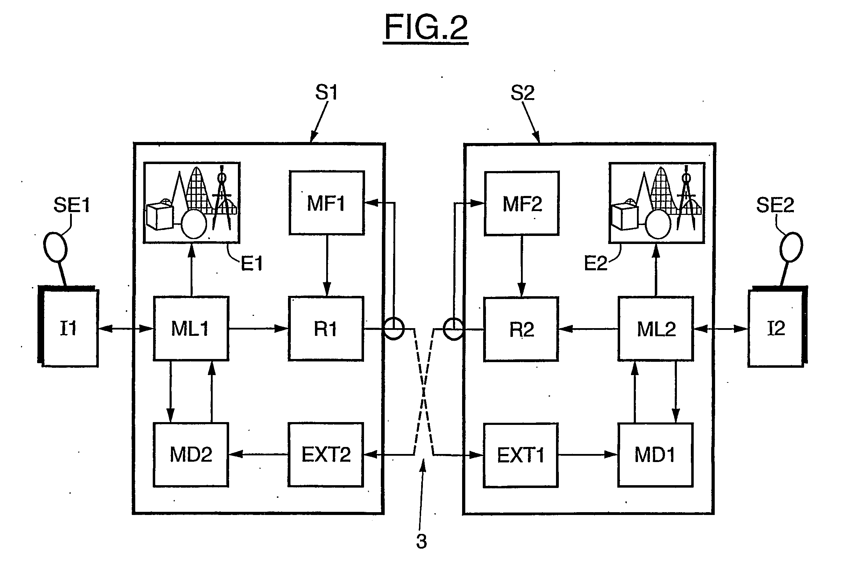

Un mode de réalisation de l'invention destiné à l'échographie est illustré sur la figure 1. Il est prévu un système de commande S1 installé par exemple dans un établissement non spécialisé en obstétrique, dans un établissement d'une ville de petite taille, ou encore dans un véhicule pour la desserte de zones rurales. Le système S2 est installé dans un établissement hospitalier spécialisé où des opérateurs hautement qualifiés sont disponibles pour réaliser les opérations d'échographie, par exemple dans un centre hospitalier régional ou universitaire. Une patiente J3 repose sur un lit ou une table T. Une sonde échographique SE est en contact avec son abdomen. Un tableau de réglage TR de paramètres de la sonde SE est installé à proximité. La sonde SE est relié au système S1 et transmet des données d'images échographiques audit système S1, et échange des données relatives à la position et aux effets exercés avec le système S1. Pour des raisons de clarté du dessin, le support de la sonde SE qui pourrait être un bras articulé n'a pas été représenté ici. Toutefois, on comprend qu'il s'agit d'un support permettant un déplacement dans l'espace selon plusieurs degrés de liberté, en général au moins six pour pouvoir prendre une position adaptée en contact avec l'abdomen de la patiente J3. Il est prévu un microphone MI3 et un haut-parleur HP3 relié au système S 1 et permettant à la patiente de converser avec l'opérateur situé à distance. Il est encore prévu une caméra CA3 orientée vers la patiente J3 et un écran vidéo EV3 permettant à la patiente de voir soit l'opérateur situé à distance, soit des images échographiques. La caméra CA3 et l'écran vidéo EV3 sont également reliés au système S1. Les systèmes S1 et S2, outre les éléments qui ont été décrits en référence aux figures 1 et 2, comprennent chacun un multiplexeur-démultiplexeur DM1 et DM2 pour permettre la transmission de données sur le réseau 3 qui peut être par exemple de type ADSL.

Du côté du système S2, l'opérateur J4 qui pourra être un médecin spécialisé en échographie manipule une poignée P3 dont la position dans l'espace va être répliquée par la sonde SE. La poignée P3 est reliée à un bras articulé BA lui-même relié à une interface I3 du genre des interfaces I1 et I2 décrites ci-dessus et comprenant un ou plusieurs actionneurs et un ou plusieurs capteurs de position et capteurs d'effort. La mesure de l'effet peut être effectuée par une mesure d'une grandeur énergétique des actionneurs, par exemple par le courant consommé encore au moyen d'une jauge de contrainte. L'interface I3 est relié au système S2.

Il est encore prévu une caméra CA4 dirigée vers l'opérateur J4 et dont les images pourront être affichées sur l'écran EV3, un microphone MI4 et un haut-parleur HP4 permettant à l'opérateur J4 de converser avec la patiente J3. Ces éléments sont reliés au système S2. Un écran vidéo EV4 de grande dimension permettra d'afficher simultanément une pluralité d'images, par exemple une image échographique, une image du visage de la patiente J3 et une image montrant le positionnement de la sonde SE sur l'abdomen de la patiente.

Comme on peut le voir sur la figure 2, un ensemble d'échographie comprend un élément SE1 de forme proche d'une sonde échographique pour l'utilisateur U 1 et une sonde échographique SE2 pour une patiente non représentée. Chaque élément SE1, SE2 est relié à une interface I1, I2 comprenant un moyen pour exercer une force sur l'élément SE1, SE2, par exemple un actionneur du genre vérin électrique, et un moyen pour mesurer la force exercée par l'utilisateur U1 et la patiente sur l'élément SE1, SE2, par exemple un capteur de couple ou encore une jauge de contrainte. L'interface I1, I2 comprendra également une carte d'acquisition reliée au moyen d'exercice d'une force et au moyen de mesure et capable d'échanger des données numériques avec un autre système numérique tel qu'un ordinateur.

Chaque interface I1, I2 est reliée à un système de commande S1, S2. Dans le cas illustré ici, les systèmes S1 et S2 sont identiques. Seul le système S1 sera décrit. Toutefois, on peut envisager des modes de réalisation dans lesquels l'un des deux systèmes est de structure simplifiée par rapport à l'autre.

De façon générale, le système S 1 peut se présenter sous la forme d'un ordinateur, du genre ordinateur personnel généralement pourvu d'au moins un micro-processeur, de mémoires rémanente et non rémanente, d'un bus de communication, de ports d'entrée et de sortie et d'un ou plusieurs logiciels stockés en mémoire et aptes à être exécutés par le micro-processeur.

Le système S1 est relié d'une part à l'interface I1 par exemple par un bus de type RS 232 et au système S2 par un réseau de communication référencé 3 dans son ensemble et qui pourra être de type synchrone par exemple RNIS ou asynchrone, de type ATM, UMTS ou encore Internet (TCP/IP). Le système S1 est situé à proximité de l'utilisateur U1, par exemple dans la même pièce. Le système S2 est situé à distance du système S1, distance qui peut aller de quelques mètres à quelques milliers de kilomètres. En d'autres termes, le système S1, l'interface I1, l'élément SE1 et l'utilisateur U1 sont disposés de façon locale tandis que le système S2, l'interface I2, l'élément SE2 et la patiente sont disposés de façon distale par rapport aux précédents.

Plus précisément, le système S 1 comprend un modèle local ML1 apte à envoyer une consigne à l'interface I1 et à recevoir de ladite interface I1 une variable mesurée par l'interface I1, par exemple la position X de l'élément SE1. La consigne peut être une variable de force ou de couple et est notée $F_e$. Le système S 1 comprend un modèle distant MD2 prévu pour estimer un état du modèle local ML2 du système S2. Le modèle distant MD2 du système S1 est apte à recevoir des données en provenance du système S2, à recevoir des données en provenance du modèle local ML1 et à émettre des données vers le modèle local ML1. Plus particulièrement, le système S1 comprend un extrapolateur EXT2 recevant des données en provenance du système S2 par l'intermédiaire du réseau de communication 3 pour traiter un message de recalage provenant du système S2 et transmettre des données de mise à jour au modèle distant MD2 en fonction du message de recalage reçu en dernier.

Le système S1 comprend un écran E1 relié au modèle local ML1 pour l'affichage de données issues du modèle local ML1, par exemple une courbe retraçant l'évolution des forces exercées et des positions des éléments SE1 et SE2.

Le système S 1 comprend un recaleur R1 recevant des données du modèle local ML1 et apte à envoyer des données de sortie à destination du système S2, en particulier à destination de l'extrapolateur EXT1 du système S2. Le recaleur R1 est apte à effectuer une préparation de données pour les émettre sous la forme d'un message de recalage qui pourra comprendre une date, la position X de l'élément SE1, la force F exercée sur l'élément SE1 à ladite date ainsi que la force exercée sur l'élément SE1 à une date antérieure.

Le système S1 comprend, en outre, un modèle fantôme MF1 qui reçoit également les messages de recalage en provenance du recaleur R1 du système S1 et qui effectue une estimation des variables d'état de l'interface I1 d'après les messages de recalage émis par le recaleur R1 et reçu par le système S2. En d'autres termes, le modèle fantôme MF1 effectue une estimation d'après les mêmes données que celles reçues par le modèle distant MD 1 du système S2. Ainsi, le modèle fantôme MF1 permet de modéliser les variables de l'interface I1 telles qu'elles sont modélisées par le système S2.

La sortie du modèle fantôme MF1 est reliée au recaleur R1 qui compare l'estimation des variables d'état provenant du modèle fantôme MF1 et les variables d'état provenant du modèle local ML1. En cas de différence supérieure à un seuil prédéterminé, le recaleur R1 émet un message de recalage destiné au modèle fantôme MF1 et à l'extrapolateur EXT1 du système S2. Ainsi, le volume de données échangé entre les systèmes S 1 et S2 est relativement réduit dans la mesure où un message de recalage n'est émis que si l'un des deux systèmes S1, S2 estime que l'autre système S2, S1 n'est plus en mesure d'estimer convenablement ces variables d'état.

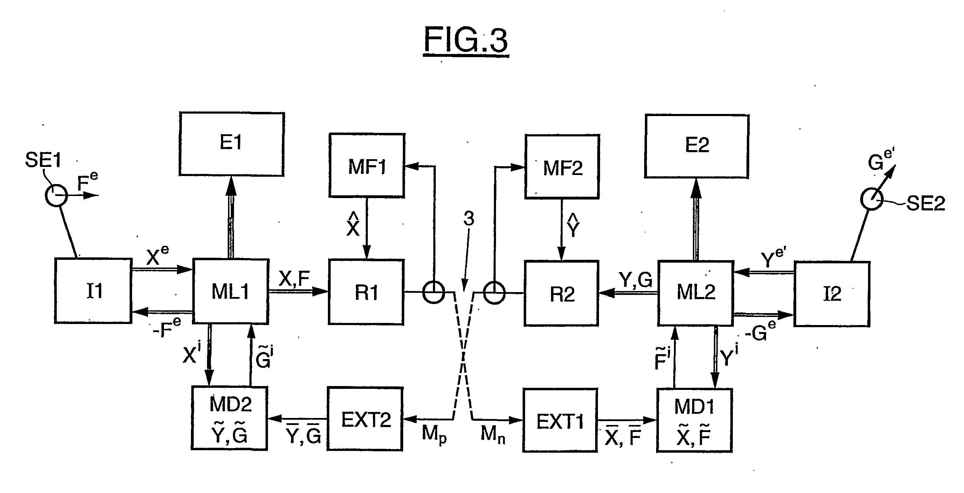

Le fonctionnement du système sera mieux compris en référence à la figure 3. Pour l'utilisateur U1, le vecteur d'état X se décompose en trois parties : $X^e$ variable située à l'interface avec l'élément SE1, $X^m$ variable interne au modèle mécanique de l'utilisateur U1 et $X^i$ variable d'interaction située à l'interface avec l'autre joueur. De façon analogue, la variable associée de force ou de couple F se décompose en : $F^e$ force exercée par l'utilisateur U1 sur l'élément SE1, $F^m$ force exercée par la pesanteur, les autres objets, d'autres joueurs éventuels, et la force $F^i$ exercée par l'utilisateur U1 sur la patiente. De manière analogue, le vecteur d'état Y de la patiente se décompose en $Y'^e$, $Y'^m$ et $Y'^i$ et le vecteur associé de force de couple G se décompose en $G'^e$, $G'^m$, et $G'^i$. L'utilisateur U1 et la patiente sont en contact virtuel. On a donc $X^i = Y^i$. La loi de l'action et de la réaction donne : $F^i + G'^i = 0$.

A chaque pas de temps, l'interface I1 capte la position $X^e_n$ et la transmet au modèle local ML1. L'interface I1 reçoit la force de consigne $F^e_n$ en provenance du modèle local ML1 et commande son ou ses actionneurs avec la force de contre-réaction $- F^e_n$. De façon analogue, l'interface I2 capte la position $Y'^e_n$ et la transmet au modèle local ML2 et reçoit la force $G'^e_n$ en provenance du modèle local ML2 commande son ou ses actionneurs avec le force de contre-réaction $- G'^e_n$.

Au début de l'instant n +1, le modèle local ML1 reçoit la position $X^e_{n+1}$ de l'interface I1, l'estimation d'interaction $\tilde{G}^i_{n+1}$ du modèle distant MD2 et les variables intrinsèques préenregistrées $F^m_{n+1}$. Le modèle local ML1 calcule la force exercée par le joueur J1 sur la patiente : $F^i_{n+1} = \tilde{G}^i_{n+1}$, la force exercée par l'utilisateur U1 sur l'élément SE1 : $F^e_{n+1} = B^{ee-1}\{X^e_{n+1} - X^e_n - A^e X_n - B^{em}F^m_{n+1} + B^{ei} \cdot \tilde{G}^i_{n+1}\}$, les matrices A et B étant celles de l'évolution dé l'utilisateur U1 avec X = AX +BF. Le modèle local ML1 calcule encore :

$$X^{m,i}_{n+1} = X^{m,i}_n + A^{m,i}X_n + B^{m,i}\begin{bmatrix} F^e_{n+1} \\ F^m_{n+1} \\ -\tilde{G}_{n+1} \end{bmatrix}$$

Le modèle local ML1 envoie $X_{n+1}$ et $F_{n+1}$ au recaleur R1, la consigne $- F^e_{n+1}$ à l'interface I1 et la variable de position $X^i_{n+1}$ au modèle distant MD2.

Le modèle fantôme MF1 s'il ne reçoit pas de message du recaleur R1 calcule $F_{n+1} = F_n + K_1$, $K_1$ étant fourni par le système S2, et l'estimation de position $F_{n+1} = (I+A)X_n + BF_{n+1}$, c'est-à-dire l'état mécanique de l'utilisateur U1 tel qu'il peut être prédit par le système S2. I est ici la matrice identité.

Sur réception d'un message de recalage $M_R = \{n, \overline{X}_n, \overline{F}_n$ et $\overline{F}_{n-1}\}$ en provenance du recaleur R1, le modèle fantôme MF1 effectue le recalage suivant : $X_n = \overline{X}_n$, $F_n = \overline{F}_n$ et $K1 = \overline{F}_n - \overline{F}_{n-1}$

Le recaleur R1 reçoit à chaque pas de temps n la variable de position $X_n$ et les variables d'effort $F_n$ et $F_{n+1}$ en provenance du modèle local ML1, et l'estimation $\hat{X}_n$ en provenance du modèle fantôme MF1. Il compare la valeur absolue de la différence entre la variable de position $X_n$ et l'estimation $\hat{X}_n$ à un seuil prédéterminé et ne fait rien si ladite valeur absolue est inférieure audit seuil. Dans le cas contraire, il compose un message de recalage $M_n = \{n, X_n, F_n, F_{n-1}\}$. Le recaleur R1 envoie le message de recalage $M_n$ au modèle fantôme MF1 pour qu'il se recale immédiatement et au modèle distant MD1 par l'intermédiaire de l'extrapolateur EXT1 du système S2 pour qu'il se recale le plus tôt possible.

L'extrapolateur EXT2 du système S1 permet de réaliser une synchronisation. En effet, le message $Mp = \{p, Y_p, G_p, G_{p-1}\}$ émis par le recaleur R2 du système S2 arrive au système S1 à un instant compris entre n et n+ 1. Toutefois, le message Mp est estampillé par la date p en provenance du système S2. L'extrapolateur EXT2 calcule $K_2 = G_p - G_{p-1}$ et recale le modèle distant MD2 en effectuant : $\overline{G}_p = G_p$ et $\overline{Y}_p = Y_p$ puis aux instants suivants et quel que soit : j = p,..., n, $\overline{G}_{j+1} = \overline{G}_j + K2$ et $\overline{Y}_{j+1} = \overline{Y}_j + C\overline{Y}_j + D\overline{G}_{j+1}$, C et D étant les matrices équivalentes aux matrices A et B pour la patiente . L'extrapolateur EXT2 transmet au modèle distant MD2 le résultat du recalage : $\overline{Y}_{n+1}$, $\overline{G}_{n+1}$ et $K_2$.

Le modèle distant MD2 du système S1 se recale sur réception d'un message en provenance de l'extrapolateur EXT2 en prenant les valeurs fournies par ledit extrapolateur EXT2 :

$$\widetilde{G}_{n+1} = \overline{G}_{n+1}, \ \widetilde{Y}_{n+1} = \overline{Y}_{n+1} \text{ et } \widetilde{K}_2 = K_2$$

Hors réception d'un tel message, et à chaque pas de temps, le modèle distant MD2 reçoit la variable dé position $X^i_{n+1}$ en provenance du modèle local ML1 et effectue un calcul prédictif :

$$\tilde{G}^{e',m'}_{n+1} = \tilde{G}^{e',m'}_{n}\tilde{K}^{e',m'}2$$

$$\tilde{G}^i_{n+1} = D^{ii-1}\left\{X^i_{n+1} - \tilde{Y}^i_n - C\tilde{Y}^i_n - D^{e'}G^{e'}_{n+1} - D^{m'}G^{m'}_{n+1}\right\}$$

$$\tilde{Y}^{e',m'}_{n+1} = \tilde{Y}^{e',m'}_n + C^{e',m'}\tilde{Y}_n + D^{e',m'}\tilde{G}_{n+1}$$

$$\tilde{Y}^i_{n+1} = X^i_{n+1}$$

Le modèle distant MD2 transmet au modèle local ML1 la prédiction de variable de position relative à la patiente : $\tilde{G}_{n+1}$

De préférence, l'extrapolateur EXT2 effectue un recalage en biseau qui permet de lisser les évolutions, voir figure 3.

Au lieu de ramener brutalement l'estimation de la variable de position Y à la variable $\overline{Y}$ calculée comme exposée ci-dessus par l'extrapolateur EXT2, le recalage est effectué en quatre étapes entre les instants n et n+4 selon le calcul suivant :

$$\text{k} = \left|\ \tilde{Y}_{n+1} := \overline{Y}_{n+1}\ \right| / \text{seuil} + 1\ ;$$

Si k = 1, alors $\tilde{Y}_{n+1} - \overline{Y}_{n+1}$

Sinon j = n,

$$\overline{G}_{j+1} = \overline{G}_j + K2$$

$$\overline{Y}_{j+1} = \overline{Y}_j + C\overline{Y}_j + D\overline{G}_{j+1}$$

$$\tilde{Y}_{j+1} := \tilde{Y}_j + C\tilde{Y}_j + D\overline{G}_{j+1}$$

$$\tilde{Y}_{j+1} = \left(\overline{Y}_{j+1} + (K-1)\tilde{Y}_{j+1}\right)/k$$

$$j := j+1$$

Si k est supérieur à 2, alors k : = k-1

Sinon on sort de la boucle et $\tilde{Y}_{j+1} = \overline{Y}_{j+1}$. Le recalage en biseau permet un fonctionnement plus doux du système, ce qui est mieux perçu par les utilisateurs et implique moins de contraintes mécaniques.

De façon plus générale, le modèle fantôme MF1 reçoit les mêmes données que le modèle distant MD1 de l'autre système et permet d'effectuer la même simulation que ledit autre système. En d'autres termes, on cherche à savoir ce que l'autre système ne sait pas dans un but de recalage. Le recaleur fonctionne en aveugle par rapport à l'autre système et permet de continuer à simuler en l'absence de données pertinentes transmises par un message de recalage en provenance de l'autre système. L'extrapolateur EXT2, en particulier dans le cas du recalage en biseau, permet de tenir compte du mouvement tel que mesuré par l'autre système pendant le délai de transmission dû au réseau de communication. Dans une variante simplifiée, on peut parfaitement concevoir que l'un des deux ou les deux systèmes est dépourvu de modèle fantôme. On peut également prévoir de faire fonctionner ensemble un nombre de systèmes supérieur à deux.

Les modèles locaux représentent les modèles mécaniques des deux utilisateurs. Les modèles distants représentent une réplication distante des modèles mécaniques locaux nécessairement approchée du fait des délais de transmission via le réseau de communication des états des modèles locaux. Les modèles fantômes représentent une copie locale approchée des modèles distants. Les modèles distants et les modèles fantômes fonctionnent tous en mode prédicteur-correcteur. Les extrapolateurs effectuent une extrapolation des messages reçus avec un certain retard pour recaler les modèles distants à la valeur de l'horloge de l'autre système. Les recaleurs évaluent la nécessité de lancer un message de recalage sur le réseau de communication dès qu'un écart trop grand apparaît entre les modèles locaux et les modèles fantômes témoins prédictifs locaux des modèles prédictifs distants. Les recaleurs permettent de limiter le nombre de messages émis à travers le réseau de communication pour éviter de l'encombrer. A l'intérieur d'un système, les échanges d'information peuvent être effectués à la cadence d'un khz. Entre les systèmes et donc par l'intermédiaire du réseau de communication, les échanges de message s'effectuent si l'un des recaleurs le considère comme nécessaire.

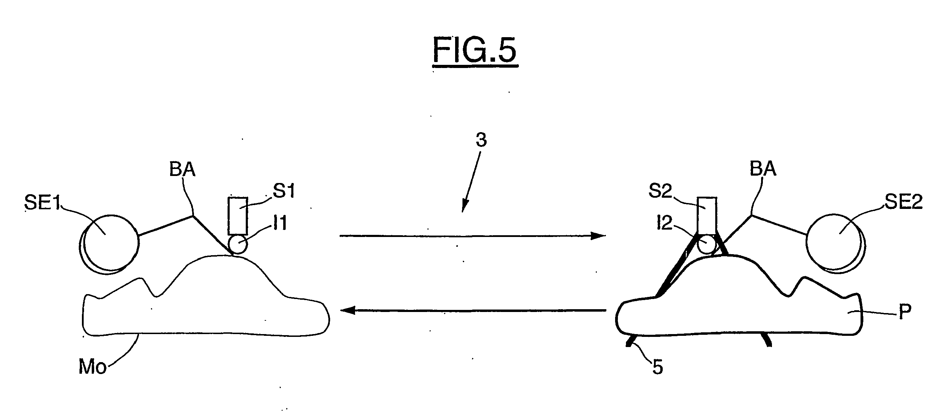

Sur la figure 5, est représenté un autre mode de réalisation particulièrement adapté à l'échographie. A proximité de la patiente P est disposée la sonde échographique SE2 supportée par le bras BA relié à l'interface 12, lui-même relié au système S2. Ces éléments sont supportés par un support 5. Dans un local situé à distance, sont disposés le système S1, l'interface I1 et un élément mobile SE1 de même forme extérieure, de même masse et de même inertie que la sonde SE2.

On a représenté en trait fin la modélisation distante Mo dans son état final, c'est-à-dire extrêmement proche de la forme de la patiente P. L'élément mobile SE1 est ici disposé dans un état de repos, dans une position relative à la modélisation distante Mo identique à la position de la sonde SE2 relativement à la patiente P et écarté de celle-là. L'élément mobile SE1 va être saisi par un praticien comme l'aurait été une véritable sonde échographique et va être descendu progressivement vers le bas. La sonde échographique SE2 suit le même déplacement que l'élément mobile SE1. Le praticien vient d'abord, en déplaçant l'élément mobile SE1, placer la sonde échographique SE2 sur la surface la plus haute de la patiente, en générale le sommet de son abdomen. La force de contre-réaction exercée par l'abdomen de la patiente P sur la sonde SE2 est recopiée à distance de façon que le praticien maniant l'élément mobile SE1 perçoive le même effet, en d'autres termes la même force de contre-réaction, que s'il maniait une sonde échographique réelle contre l'abdomen d'une patiente.

A partir du sommet de l'abdomen de la patiente P, le praticien maniant l'élément mobile SE1 en le déplaçant à la fois verticalement et horizontalement, va découvrir progressivement le contour de la patiente P, éventuellement en s'aidant d'un écran de visualisation tel que l'écran EV4 illustré sur la figure 1. Le praticien en manipulant l'élément mobile SE1 va faire décrire l'abdomen de la patiente P à la sonde SE2 qui en réplique le mouvement. La force exercée par l'abdomen de la patiente sur la sonde SE2 est répliquée par l'élément mobile SE1.

Les positions de l'élément mobile SE1 et de la sonde SE2 étant sensiblement identiques, l'élément mobile SE1 est positionné de façon sensiblement identique à la position qu'aurait une sonde réelle parcourant l'abdomen de la même patiente. Le système S1 enregistre les différentes positions de l'élément mobile SE1, ce qui permet à partir d'un nombre de points de mesure suffisant, d'effectuer une reconstruction tridimensionnelle de la surface extérieure de l'abdomen de la patiente, et donc d'obtenir la modélisation distante Mo.

On pourra prévoir une plage de force de contre-réaction dans laquelle le système S1 considérera que l'élément mobile SE1 est dans une position correspondante à la surface de l'abdomen de la patiente. Au-dessus de la borne supérieure de la plage, on considérera que l'élément mobile SE1 est trop enfoncé dans l'abdomen. En dessous de la borne inférieure de la plage, on considérera qu'il n'y a pas contact et que l'élément mobile est situé hors de la surface extérieure de l'abdomen de la patiente P.

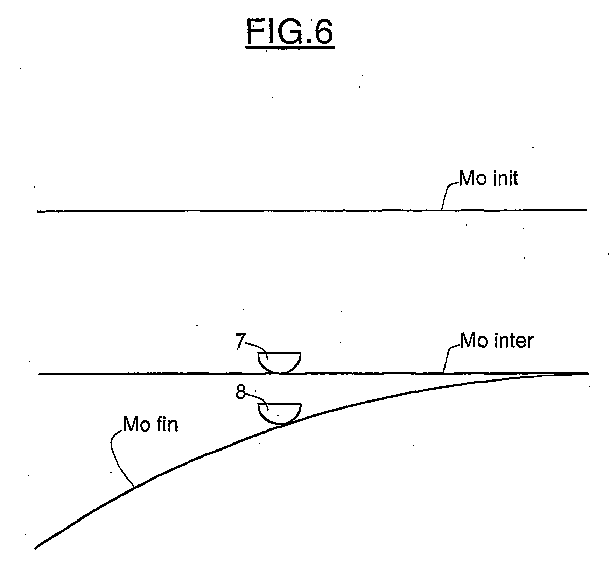

La figure 6 est un schéma illustrant les étapes de génération de la modélisation distante. Tout d'abord, la modélisation distante se présente sous la forme d'une modélisation initiale $Mo_{inter}$, qui est une surface plane maillée, vue ici en coupe. Puis, le praticien en manipulant l'élément mobile SE1 positionne la sonde échographique SE2 sur le sommet de l'abdomen de la patiente P. La modélisation initiale $Mo_{init}$ devient alors une modélisation intermédiaire $Mo_{inter}$, toujours sous la forme d'une surface plane, mais tangente au sommet de l'abdomen de la patiente. En d'autres termes, les coordonnées verticales des points de la modélisation ont été diminuées de la distance séparant la modélisation initiale $Mo_{init}$ du sommet de l'abdomen de la patiente, les modélisations initiale $Mo_{init}$ et intermédiaire $Mo_{inter}$ étant des plans horizontaux.

Le praticien décale alors la sonde SE1 horizontalement et l'amène, par exemple dans la position 7 affleurant la modélisation intermédiaire $Mo_{inter}$, puis amène l'élément mobile dans la position 8, de sorte que la sonde échographique SE2 est en contact avec l'abdomen de la patiente P. Dès que la force de contre-réaction exercée par l'abdomen de la patiente P sur la sonde SE2 dépasse la borne inférieure de la plage et est transmise au système S1, ledit système S1 enregistre la position comme appartenant à la surface supérieure de l'abdomen de la patiente P. En pratique, le praticien pourra effectuer des mouvements courbes de façon que la sonde SE2 suive la surface supérieure de l'abdomen de la patiente P, et ce à la fois pour le confort de la patiente et la rapidité du procédé. En outre, lors de tout déplacement de l'élément mobile SE1 au-delà de la modélisation, on peut prévoir qu'une légère force de contre-réaction soit exercée par l'élément mobile SE1 sur les mains du praticien de façon que ledit franchissement lui soit perceptible. Ladite force sera commandée par le système S 1 et appliquée par l'interface I1, et pourra être réglable tout en restant inférieure à la borne inférieure de ladite plage. Ladite force pourra comprendre une partie constante analogue à un frottement sec et une partie variable proportionnelle à la vitesse du déplacement de l'élément mobile SE1.

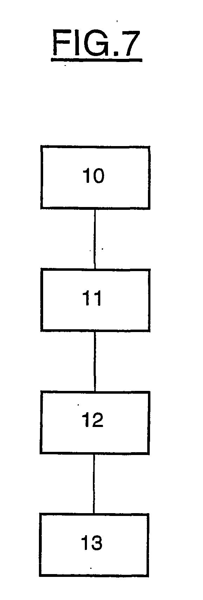

Sur la figure 7, sont illustrées de façon schématique, les différentes étapes du procédé. A l'étape 10 d'initialisation, la sonde SE2 et l'élément mobile SE1 se placent dans une position d'attente ou de repos, permettant à la patiente P de s'installer sur une table d'examen ou de quitter une telle table. A l'étape 11, le praticien commande l'approche de la sonde SE2 vers le sommet de l'abdomen de la patiente P. A l'étape 12, a lieu le contact entre la sonde SE2 et le sommet de l'abdomen de la patiente, l'enregistrement par le système S1 des coordonnées du premier point de contact et le déplacement de la modélisation distante Mo de la modélisation distante initiale $Mo_{init}$ à la modélisation distante intermédiaire $Mo_{inter}$. A l'étape 13, le praticien fait parcourir à la sonde SE2 la surface supérieure de l'abdomen de la patiente. A chaque fois qu'un point de coordonnées déterminé est considéré comme appartenant à la surface supérieure de l'abdomen de la patiente P, la modélisation distante intermédiaire $Mo_{inter}$ est déformée maille par maille ou noeud par noeud pour épouser la forme de la surface supérieure de l'abdomen de la patiente P. On parvient ainsi progressivement à la modélisation distante finale $Mo_{fin}$.

La modélisation distante Mo peut comprendre une maille de taille variable ou fixe. On peut mettre en oeuvre éventuellement une resegmentation afin d'affiner la modélisation dans certaines zones.

A l'issue de cet étalonnage, le système S1 stocke dans une mémoire la modélisation distante finale, ce qui lui permet de réagir avec un temps de réponse extrêmement bref, même si le réseau de transmission 3 pâtit de délais de transmission élevés. Ultérieurement, lorsque le praticien déplacera l'élément mobile SE1 en traversant la modélisation distante finale, le système S 1 appliquera une force à l'élément mobile SE1, simulant la force de contre-réaction de l'abdomen de la patiente P sur la sonde SE2. En y ajoutant éventuellement un terme de frottement afin d'éviter des mouvements trop rapides de l'élément mobile SE1 et donc de la sonde échographique SE2.

L'invention met donc en oeuvre une déformation d'un maillage géométrique sous le geste du praticien utilisateur. Le télé-étalonnage du mannequin virtuel ou modélisation distante, s'effectue par un balayage intuitif du praticien utilisateur afin de faire tendre le maillage de départ ou modélisation distante initiale vers la forme de la patiente dont la corpulence et notamment le stade de la grossesse sont extrêmement variables d'une personne à une autre. On peut ainsi palper une personne ou l'objet situé à distance d'un poste maître, ce qui permet d'approcher une forme tridimensionnelle et d'appréhender la forme de la personne ou de l'objet, la présence d'une caméra n'étant pas indispensable. Dans le cadre d'une application utilisant déjà un système à retour d'effort, la méthode proposée permet la reconstruction à distance de la forme tridimensionnelle sans recourir aux méthodes antérieures comme les relevés lasers ou la stéréophotographie.

**Revendications**

1. Procédé de reconstruction à distance d'une surface, dans lequel :

   - un système local envoie à un système distant une information de position d'un élément mobile dudit système local, l'élément mobile du système local répliquant la position de l'élément mobile du système distant, le système distant comprenant une modélisation distante de la surface,
   - un opérateur distant déplace l'élément mobile du système distant, et lorsque l'élément mobile du système local entre en contact avec ladite surface, ladite modélisation distante est modifiée pour chaque point de contact entre l'élément mobile du système local et ladite surface de façon que la modélisation distante se rapproche de ladite surface.

2. Procédé selon la revendication 1, dans lequel la modélisation distante est initialement une surface plane maillée.

3. Procédé selon la revendication 1 ou 2, dans lequel la modélisation distante est rapprochée dans son ensemble de ladite surface selon un mouvement de translation normale à une portion de ladite surface et normale à une portion de la modélisation distante, jusqu'à un premier point de contact de l'élément mobile du système local avec ladite surface.

4. Procédé selon l'une quelconque des revendications précédentes, dans lequel un point de contact est un point de l'espace commun à l'élément mobile du système local et à ladite surface et tel que l'élément mobile du système local exerce sur ladite surface une force prédéterminée.

5. Procédé selon l'une quelconque des revendications précédentes, dans lequel une modélisation distante comprend une pluralité de noeuds, un noeud est approché à ladite surface par translation selon un axe.

6. Procédé selon l'une quelconque des revendications précédentes, dans lequel la force de réaction exercée par ladite surface sur l'élément mobile du système local est répliquée par l'élément mobile du système distant de façon que l'opérateur perçoive ladite force de réaction et puisse appréhender ladite surface.

7. Procédé selon l'une quelconque des revendications précédentes, dans lequel, lors d'une phase d'initialisation, l'élément mobile du système distant réplique la position de l'élément mobile du système local.

8. Procédé selon l'une quelconque des revendications précédentes, dans lequel l'élément mobile du système distant exerce une force de freinage lorsque l'élément mobile du système local est déplacé vers ladite surface dans une partie de l'espace comprise entre la modélisation distante et ladite surface.

9. Système de reconstruction à distance d'une surface, **caractérisé par le fait qu'**il comprend un système local (S2) pourvu d'un élément mobile (SE2) apte à entrer en contact avec ladite surface à reconstruire, et un système distant (S1) pourvu d'un élément mobile (SE1) apte à être manipulé par un opérateur, une modélisation distante (Mo) de la surface, et d'un moyen pour modifier ladite modélisation distante pour chaque point de contact entre l'élément mobile du système local et ladite surface lorsque l'élément mobile du système local entre en contact avec ladite surface, de façon que la modélisation distante se rapproche de ladite surface, le système local étant pourvu d'un moyen pour envoyer au système distant une information de position de l'élément mobile dudit système local, et d'un moyen pour répliquer la position de l'élément mobile du système distant.

10. Programme d'ordinateur comprenant des moyens de code-programme pour mettre en oeuvre les étapes du procédé selon l'une quelconque des revendications 1 à 8, lorsque ledit programme fonctionne sur un ordinateur.

**Claims**

1. Method for remote reconstruction of a surface, in which:

   - a local system sends information on the position of a mobile element of said local system to a remote system, the mobile element of the local system replicating the position of the mobile element of the remote system, the remote system comprising a remote modelling of the surface,
- a remote operator displaces the mobile element of the remote system, and when the mobile element of the local system comes into contact with said surface, said remote modelling is modified for each point of contact between the mobile element of the local system and said surface so that the remote modelling approximates said surface.

2. Method according to Claim 1, in which the remote modelling is initially a meshed flat surface.

3. Method according to Claim 1 or 2, in which the remote modelling overall approximates said surface along a translational movement normal to a portion of said surface and normal to a portion of the remote modelling, up to a first point of contact of the mobile element of the local system with said surface.

4. Method according to any one of the preceding claims, in which a point of contact is a point in space common to the mobile element of the local system and to said surface and such that the mobile element of the local system exerts a predetermined force on said surface.

5. Method according to any one of the preceding claims, in which a remote modelling comprises a plurality of nodes, one node approximates said surface by translation along an axis.

6. Method according to any one of the preceding claims, in which the reaction force exerted by said surface on the mobile element of the local system is replicated by the mobile element of the remote system so that the operator experiences said reaction force and is able to apprehend said surface.

7. Method according to any one of the preceding claims, in which, during an initialization phase, the mobile element of the remote system replicates the position of the mobile element of the local system.

8. Method according to any one of the preceding claims, in which the mobile element of the remote system exerts a braking force when the mobile element of the local system is displaced towards said surface in a part of the space contained between the remote modelling and said surface.

9. Ssystem for remote reconstruction of a surface, **characterized in that** it comprises a local system (S2) provided with a mobile element (SE2) capable of coming into contact with said surface to be reconstructed, and a remote system (S1) provided with a mobile element (SE1) capable of being manipulated by an operator, a remote modelling (Mo) of the surface, and a means for modifying said remote modelling for each point of contact between the mobile element of the local system and said surface when the mobile element of the local system comes into contact with said surface, so that the remote modelling approximates said surface, the local system being provided with a means for sending information on the position of the mobile element of said local system to the remote system, and with a means for replicating the position of the mobile element of the remote system.

10. Computer program comprising program code means for implementing the steps of the method according to any one of Claims 1 to 8, when said program runs on a

**Patentansprüche**

1. Verfahren zur Fern-Rekonstruktion einer Fläche, bei dem:

- ein lokales System an ein fernes System eine Positionsinformation eines beweglichen Elements des lokalen Systems sendet, wobei das bewegliche Element des lokalen Systems die Position des beweglichen Elements des fernen Systems nachbildet, wobei das ferne System eine ferne Modellierung der Fläche enthält;
- ein ferner Operator das bewegliche Element des fernen Systems verschiebt, und wenn das bewegliche Element des lokalen Systems mit der Fläche in Kontakt kommt, die ferne Modellierung für jeden Kontaktpunkt zwischen dem beweglichen Element des lokalen Systems und der Fläche so verändert wird, dass die ferne Modellierung sich der Fläche annähert.

2. Verfahren nach Anspruch 1, bei dem die ferne Modellierung ursprünglich eine ebene vermaschte Fläche ist.

3. Verfahren nach Anspruch 1 oder 2, bei dem die ferne Modellierung in ihrer Gesamtheit der Fläche gemäß einer Translationsbewegung orthogonal zu einem Abschnitt der Fläche und orthogonal zu einem Abschnitt der fernen Modellierung angenähert wird, bis zu einem ersten Kontaktpunkt des beweglichen Elements des lokalen Systems mit der Fläche.

4. Verfahren nach einem der vorhergehenden Ansprüche, bei dem ein Kontaktpunkt ein Punkt des dem beweglichen Elements des lokalen Systems und der Fläche gemeinsamen Raums und derart ist, dass das bewegliche Element des lokalen Systems auf die Fläche eine vorbestimmte Kraft ausübt.

5. Verfahren nach einem der vorhergehenden Ansprüche, bei dem eine ferne Modellierung mehrere Knoten aufweist, wobei ein Knoten der Fläche durch Translationsverschiebung gemäß einer Achse angenähert wird.

6. Verfahren nach einem der vorhergehenden Ansprüche, bei dem die von der Fläche auf das bewegliche Element des lokalen Systems ausgeübte Reaktionskraft vom beweglichen Element des fernen Systems nachgebildet wird, damit der Operator die Kraft bemerkt und die Fläche erfassen kann.

7. Verfahren nach einem der vorhergehenden Ansprüche, bei dem in einer Initialisierungsphase das bewegliche Element des fernen Systems die Position des beweglichen Elements des lokalen Systems nachbildet.

8. Verfahren nach einem der vorhergehenden Ansprüche, bei dem das bewegliche Element des fernen Systems eine Bremskraft ausübt, wenn das bewegliche Element des lokalen Systems zur Fläche in einem Bereich des Raums verschoben wird, der zwischen der fernen Modellierung und der Fläche liegt.

9. System zur Fern-Rekonstruktion einer Fläche, **dadurch gekennzeichnet, dass** es ein lokales System (S2), das mit einem beweglichen Element (SE2) versehen ist, das in der Lage ist, mit der zu rekonstruierenden Fläche in Kontakt zu kommen, und ein fernes Systems (S1) aufweist, das mit einem beweglichen Element (SE1), das von einem Operator manipuliert werden kann, einer fernen Modellierung (Mo) der Fläche, und mit einem Mittel zur Veränderung der fernen Modellierung für jeden Kontaktpunkt zwischen dem beweglichen Element des lokalen Systems und der Fläche versehen ist, wenn das bewegliche Element des lokalen Systems mit der Fläche in Kontakt kommt, so dass die ferne Modellierung sich der Fläche annähert, wobei das lokale System mit einem Mittel, um an das ferne System eine Positionsinformation des beweglichen Elements des lokalen Systems zu senden, und mit einem Mittel versehen ist, um die Position des beweglichen Elements des fernen Systems nachzubilden.

10. Computerprogramm, das Code-Programm-Mittel aufweist, um die Schritte des Verfahrens nach einem der Ansprüche 1 bis 8 durchzuführen, wenn das Programm auf einem Computer läuft.

# FIG.1

EP 1 370 188 B1

# FIG.2

EP 1 370 188 B1

FIG.3

FIG.4

MD2 après recalage $\tilde{Y}$

EXT2 $\overline{Y}$

MD2 sans recalage

Recalages successifs

> seuil

Réception du message M

Emission du message M

Y

t

p      n      n+1      n+2      n+3      n+3

EP 1 370 188 B1

# FIG.5

EP 1 370 188 B1

# FIG.6

Mo init

7

Mo inter

8

Mo fin

# FIG.7